(19) **Europäisches Patentamt**

**European Patent Office**

**Office européen des brevets**

(11) **EP 1 637 884 A1**

(12) **EUROPEAN PATENT APPLICATION**
published in accordance with Art. 158(3) EPC

(43) Date of publication:
22.03.2006 Bulletin 2006/12

(51) Int Cl.:
*G01N 33/543* (1985.01)     *G01N 15/02* (1968.09)
*G01N 15/12* (1985.01)

(21) Application number: 04746066.2

(22) Date of filing: 11.06.2004

(86) International application number:
PCT/JP2004/008553

(87) International publication number:
WO 2004/111649 (23.12.2004 Gazette 2004/52)

(84) Designated Contracting States:
CH DE ES FR GB IT LI

(30) Priority: 16.06.2003 JP 2003170425

(71) Applicant: Pulse-Immunotech Corporation
Hachioji-shi, Tokyo 192-0982 (JP)

(72) Inventors:
• KARUBE, Isao
Yokohama-shi
Kanagawa 2250002 (JP)

• IWATA, Keisuke
Saitama 3460011 (JP)

(74) Representative: Grünecker, Kinkeldey,
Stockmair & Schwanhäusser
Anwaltssozietät
Maximilianstrasse 58
80538 München (DE)

(54) **METHOD FOR MEASURING SUBSTANCE HAVING AFFINITY**

(57)     The binding reaction between an affinity substance to be measured and a binding partner having binding affinity for the affinity substance is measured based on agglutination reactions. Carrier particles bound to the binding partner are allowed to bind to the affinity substance in an electric field. Evaluation is achieved by counting the level of agglutinated carrier particles using three-dimensional particle information of the particles as an indicator.

The use of three-dimensional information as an indicator enables the presence of a biologically specific reactive substance to be detected or measured in a manner that is more convenient and rapid, and has a higher sensitivity than the conventional measurement methods.

EP 1 637 884 A1

**Description**

Technical Field

The present invention relates to methods and devices for measuring substances having affinity (also referred to as "affinity substances") using agglutination reactions of carrier particles.

Background Art

Conventional methods for detecting or measuring the presence of specific biological reactive substances include, for example, enzyme immunoassays and radioimmunoassays. These are highly sensitive and accurate methods. However, their reagents are unstable because enzymes or radioisotopes are used as labels. Furthermore, these assays that use radioisotopes require meticulous attention to detail and technical skills because there are regulations for radioisotope storage and preservation. Thus, there has been a need for more convenient measurement methods. Furthermore, since these methods require a relatively long time for measurement, they cannot be applied for urgent tests. Under these circumstances, rapid and highly sensitive measurement methods started to be extensively studied.

Since 1970, analysis methods that use agglutination of carrier particles as an indicator for measuring immunological reactions have been put into practical use. In these methods, quantitative analysis is enabled by optical measurement of the degree of carrier particle agglutination. The optical methods that use latex particles as a carrier particle for measuring immunological particle agglutination reactions are called latex agglutination turbidimetry. In general, the reaction temperature in these analysis methods ranges from 37 to 45°C, and specific agglutination reactions proceed upon mixing with a stirring impeller or such. Since the time required for measurement (reaction) ranges from about 10 to 20 minutes, these methods are more rapid than enzyme immunoassays or radioimmunoassays. However, these methods are said to be inferior to enzyme immunoassays or such in sensitivity and measurement range.

Methods for determining the particle size distribution in latex agglutination methods are also known (Non-patent Document 1, Cambiaso et al., J. Immunol. Methods 18, 33, 1977; Non-patent Document 2, Matsuzawa et al., Kagaku to kogyo (Chemistry and Chemical Industry), Vol. 36, No. 4, 1982). In latex agglutination turbidimetry, light transmittance through particle suspensions is determined by measuring the state and the number of individually dispersed particles by methods that determine particle size distribution. In the report of Cambiaso et al., an antigen was reacted with a reagent of antibody-bound latex particles (0.8 $\mu$m diameter) at 37°C for 20 minutes. The particles were counted after the reaction and the antigen was quantified based on the level of decrease in the number of particles due to agglutination. The number of particles was determined using a counter that is based on the principle of laser light scattering.

Meanwhile, Matsuzawa et al. incubated an antigen with a reagent of antibody-bound latex particles (1 $\mu$m diameter) for 6 hours. After the reaction, mean particle volume was determined by an electric resistance method to quantify the antigen. However, only the PAMIA system (SYSMEX CORPORATION), which uses a laser scattering sheath flow method, has been put into practical use and is widely used. PAMIA uses latex particles that have a diameter of 0.78 $\mu$m. Immunoassay is carried out by counting latex particles after a 15-minute reaction at 45°C. PAMIA is more sensitive than latex agglutination turbidimetry. However, PAMIA is said to be inferior in sensitivity when compared to high sensitivity immunoassay methods such as radioimmunoassays (RIA) and enzyme immunoassays (EIA).

In general, latex agglutination turbidimetry uses latex particles that have a diameter of 0.05 to 0.6 $\mu$m. When such small particles are used, methods for analyzing particle size distribution in latex agglutination are easily affected by substances that interfere with measurement. For example, lipids, proteins, blood cell components, and such coexist in body fluids such as blood and urine. These coexisting substances are indistinguishable from carrier particles, and may lead to inaccurate counting of carrier particles. Hence, relatively large particles have been used to avoid the impact of interfering substances of measurement. In contrast, agglutination reactions hardly take place when particles having a diameter of about 1 $\mu$m, such as those in Matsuzawa et al. are used. This is the reason why latex particles with a diameter of about 0.8 $\mu$m have been so far used. The diameter of the aperture (small hole) that Matsuzawa et al. used to measure mean particle volumes was 30 $\mu$m. Apertures of this size are more susceptible to clogging. However, 0.8- to 1-$\mu$m particles cannot be detected when the aperture diameter is greater than 30 $\mu$m.

A method that applies an alternating voltage to a reaction system to accelerate biologically specific agglutination reactions and allows resulting agglutinates to be readily detected is also known (Patent Document 1, Japanese Patent Application Kokai Publication No. (JP-A) H7-83928 (unexamined, published Japanese patent application). This method uses biologically specific agglutination reactions of carrier particles for detecting or measuring the presence of biologically specific reactive substances, and comprises applying an alternating voltage to a reaction system to provide an electric field strength of 5 to 50 V/mm in the presence of salt (10 mM or higher).

When placed in an electric field, carrier particles are linearly aligned along the electric field (pearl chain formation). The linearly aligned carrier particles re-disperse when the electric field is removed. In the presence of a biologically specific reactive substance during pearl chain formation, the carrier particles do not re-disperse and pearl chain-like particles are found even after the electric field is removed. The above-described measurement method is based on this phenomenon. Specifically, reactions of biologically specific reactive substances are accelerated in an electric field. The reaction products can be detected by allowing carrier particles to re-disperse after removal of the electric field.

Disclosure of the Invention

In the above-described known techniques, the reaction of a specific biological reactive substance is detected using the agglutination of carrier particles as an indicator. The agglutination of carrier particles is detected using two-dimensional graphic analysis data. Specifically, electrodes are first attached to a glass slide at fixed intervals, and a reaction solution mixture containing reagents and a sample is dropped over the electrode intervals (reaction vessel), which are then covered with a cover glass. An electric field is supplied by applying a voltage to the electrodes. After the electric field is removed, agglutinates of the carrier particles in the reaction solution between electrodes are observed under a microscope or such.

However, such experimental operations are complex, thus impeding mechanization. Furthermore, it is difficult to maintain high sensitivity and reproducibility in methods that involve microscopic observation of glass slides. For example, only a small amount of reaction solution can be placed in a limited space like a glass slide. That is, limitations on both the sample and the number of carrier particles make it difficult to expect sufficient measurement sensitivity. Furthermore, advanced techniques are required for small quantities of reaction solution to maintain reaction conditions such as reaction temperature and the quantitative ratio of sample to carrier particle. Thus, it is difficult to maintain high levels of reproducibility.

The detection of agglutinated particles by conventional methods was also problematic. In conventional methods, agglutinated particles were counted based on the graphic information obtained through microscopic observation. In other words, agglutinated particles were observed based on their two-dimensional information. However, it has been shown that due to various factors, two-dimensional information does not always enable correct detection of agglutinates.

For example, the size of a pearl chain-like agglutinate can be evaluated accurately only when the agglutinate is observed from a direction perpendicular to the longitudinal direction of pearl chain. When observed from along the longitudinal direction of pearl chain, the pearl chain-like agglutinate appears nearly the same size as an unagglutinated particle. Even for particles that do not form agglutinates, there is a possibility that they may be counted as agglutinates if placed in a positional relationship that makes them seem overlapping. Two-dimensional information represents only the shape of particles recognizable from a particular direction. Accordingly, in cases where the particle shape varies depending on the direction of observation, particle size cannot be accurately evaluated based on two-dimensional information. Thus, the procedure for counting particles based on two-dimensional information has become a limiting factor in measurement accuracy.

An objective of the present invention is to overcome these problems. A more specific objective is to provide measurement methods that allow easy mechanization and simple maintenance of measurement accuracy.

The present inventors intensively studied means for measuring agglutinates of carrier particles to resolve the problems described above. As a result, the inventors discovered that measurement accuracy is improved by counting particles or agglutinates using three-dimensional information as an indicator, thereby completing the present invention. Specifically, the present invention relates to the following measurement methods and devices:

[1] a method for measuring an affinity substance, which comprises the steps of:

(1) mixing carrier particles with an affinity substance to be measured and applying a voltage pulse, wherein the carrier particles are bound to a binding partner having an activity to bind to the affinity substance; or
(1') mixing carrier particles with an agglutination reagent component and an affinity substance to be measured, and applying a voltage pulse, wherein the carrier particles are bound to a binding partner having an activity to bind to the affinity substance, and wherein the affinity substance inhibits agglutination of the carrier particles caused by the agglutination reagent;
(2) counting agglutinates of carrier particles formed upon the binding of the affinity substance to be measured, or unagglutinated carrier particles which did not bound to the affinity substance, or both, based on their three-dimensional information as an indicator after step (1); or
(2') counting agglutinates of carrier particles formed upon the binding of the agglutination reagent, or carrier particles whose agglutination was inhibited through the binding of the affinity substance to be measured, or both based on their three-dimensional information as an indicator after step (1'); and
(3) determining the level of the substance to be measured based on either or both of the level of agglutinate formation and the level of unagglutinated carrier particles after step (2) or (2');

[2] the method of claim 1, wherein the three-dimensional information of agglutinates or carrier particles is physically measured in step (2) or (2');

[3] the method of claim 2, wherein the method of physically measuring the three-dimensional information is a method selected from the group consisting of electric resistance method, laser diffraction/scattering method, and three-dimensional image analysis method;

[4] the method of claim 1, wherein the voltage pulse is an alternating current voltage pulse;

[5] the method of claim 1, which comprises counting the carrier particles after the electric field is removed in step (2) or (2');

[6] the method of claim 5, which further comprises the step of diluting the carrier particles after the electric field is removed in step (2) or (2');

[7] the method of claim 1, which comprises applying voltage pulses several times;

[8] the method of claim 7, which comprises the step of applying voltage pulses, dispersing the carrier particles, and applying voltage pulses again;

[9] the method of claim 7, wherein the voltage pulses are applied from different directions;

[10] the method of claim 1, wherein the mean particle size of carrier particles is 1 $\mu$m or greater;

[11] the method of claim 10, wherein the mean particle size of carrier particles is in the range of 1 to 20 $\mu$m;

[12] a device for measuring an affinity substance, which comprises:

(a) a space containing carrier particles and an affinity substance to be measured, wherein the carrier particles are bound to a binding partner having an activity to bind to the affinity substance;

(b) electrodes for applying a voltage pulse to the carrier particles in the space; and

(c) a device for counting agglutinates formed through agglutination of carrier particles, or unagglutinated carrier particles in the space, or both, using their three-dimensional information as an indicator;

[13] the device of claim 12, wherein the device of (c) is a means for physically measuring three-dimensional information;

[14] the device of claim 13, wherein the device for physically measuring the three-dimensional information is a means for physically measuring three-dimensional information using a method selected from the group consisting of electric resistance method, laser diffraction/scattering method, and three-dimensional image analysis method;

[15] the device of claim 12, which comprises at least two pairs of electrodes for applying the voltage pulse; and

[16] the device of claim 12, which comprises a means for moving the electrodes to apply the voltage pulse and which can supply an electric field from different directions with respect to the space.

Herein, "affinity substance and binding partner having an activity to bind to the affinity substance" include every possible combination of substances that can participate in a binding reaction. Specifically, when one substance binds to another substance, one is the affinity substance and the other is the binding partner. The affinity substances and binding partners of the present invention may be natural substances or artificially synthesized compounds. The affinity substances and binding partners may be purified substances or substances containing impurities. Further, the affinity substances and binding partners may exist on cellular or viral surface.

Binding reactions between the affinity substances and binding partners of the present invention include, for example, the reactions listed below. Substances that participate in these reactions can either be an affinity substance or a binding partner of the present invention. Reaction between an antibody and an antigen or a hapten (immunological reaction); hybridization between nucleic acids having complementary nucleotide sequences;

reaction between a lectin and its receptor;

reaction between a lectin and a sugar chain;

reaction between a ligand and its receptor;

reaction between DNA and a transcription regulatory factor.

Among the above-listed binding reactions, a preferred binding reaction of the present invention can be, for example, an immunological reaction. Antigens participating in immunological reactions include the substances listed below. These antigens include not only antigen molecules themselves but also fragments thereof, and those that are present on cell surface. These substances are only examples of antigenic substances and needless to say, the present invention is also applicable to other antigenic substances. For example, any antigenic substance that can be measured based on an immunological agglutination reaction using latex or blood cell as a carrier can be used as an affinity substance of the present invention.

Tumor markers:

AFP, CEA, CA19-9, PSA, etc.

Markers of the coagulation-fibrinolytic system:

protein C, protein S, antithrombin (AT) III, FDP, FDP-D-dimer, etc.

Infection markers:

CRP, ASO, HBs antigen, etc.

Hormones:

thyroid-stimulating hormone (TSH), prolactin, insulin, etc.

Tissue components:

myoglobin, myosin, hemoglobin, etc.

Others:

nucleic acids such as DNA.

Either an antigenic substance or an antibody recognizing the substance may be used as the affinity substance and the other as the binding partner. Herein, the affinity substance refers to a target substance to be measured. On the other hand, the binding partner refers to a substance that can be used as a probe to measure the affinity substance and has an activity to bind to the affinity substance. Thus, an antibody can be used as the binding partner when an antigen is measured. Conversely, an antibody recognizing an antigen can be used as the binding partner in the measurement of the antibody. For example, any antibody that can be measured based on an immunological agglutination reaction using latex or blood cell as a carrier can be used as an affinity substance of the present invention. Antibodies against HBs (surface antigen of hepatitis B virus), HBc (core antigen of hepatitis B virus), HCV (hepatitis C), HIV (AIDS virus), TP (syphilis), and such have been measured using immunological agglutination reactions.

Several reaction principles are known to use agglutination of carrier particles as an indicator for measuring the reaction between an affinity substance and a binding partner. Any of these reaction principles can be applied to the present invention. Examples of a measurement principle that uses agglutination of carrier particles as an indicator and applies the reaction between an affinity substance and a binding partner are described below.

Direct agglutination reaction:

The agglutination of carrier particles which results from the reaction between a target substance of measurement and its binding partner present on the carrier particles is detected. This principle is applicable, for example, to cases where an antigen molecule is measured using an antibody as the binding partner. Alternatively, the principle is also applicable when an antibody is measured as the affinity substance by using agglutination of antigen-bound carrier particles as an indicator. In general, the level of agglutination is directly proportional to the amount of affinity substance to be measured in a direct agglutination reaction. Specifically, the higher the level of agglutinate formation, the higher the level (namely concentration) of an affinity substance is. Conversely, when the level of unagglutinated carrier particles is high, the level (namely concentration) of an affinity substance is low.

Agglutination inhibition reaction:

A low-molecular-weight antigen called "hapten" hardly forms the antigen-mediated cross-linking structure required for the agglutination of carrier particles. Therefore, haptens cannot be detected based on the principle of direct agglutination reaction. In this case, it is possible to use the agglutination reaction that results from the binding of an antibody on carrier particles to a polyhapten that comprises two or more hapten molecules or fragments comprising the epitope. A polyhapten can crosslink two or more antibody molecules and agglutinate carrier particles. However, in the presence of a hapten, the reaction between a polyhapten and an antibody is inhibited and as a result, the agglutination of carrier particles is inhibited. The level of agglutination inhibition is directly proportional to the presence of hapten. Specifically, the amount of a target substance of measurement is inversely proportional to the level of agglutination reaction. Specifically, the level (i.e., concentration) of an affinity substance is low when the level of agglutinate formation is high. Conversely, the higher the level of unagglutinated carrier particles, the higher the level (i.e., concentration) of an affinity substance is.

Target antigens of measurement that are classified as haptens include the following components.

Hormones:

estrogen, estradiol

Drugs:

Theophylline.

In the present invention, measuring a hapten based on the principle of agglutination inhibition reaction requires a component that allows the agglutination of carrier particles bound to an anti-hapten antibody. Herein, a component that allows the agglutination of carrier particles bound to an anti-hapten antibody is referred to as an "agglutination reagent". An agglutination reagent is defined as a reagent that has specific affinity for an antibody as well as activity of crosslinking carrier particles via antibody binding. The polyhapten described above can be used as an agglutination reagent in hapten measurements.

In both the direct agglutination reaction and the agglutination inhibition reaction, a standard curve or regression equation may be prepared by measuring standard samples containing a predetermined concentration of affinity substance using the same reaction system, and measuring the level of agglutinates or unagglutinated carrier particles. The level of affinity substance in a sample can be determined either from the level of agglutinate formation or the level of unagglutinated carrier particles determined in a sample measurement, using the standard curve or regression equation.

The binding partners of the present invention are used to bind carrier particles. The carrier particles of the present invention include latex particle, kaolin, colloidal gold, erythrocyte, gelatin, liposome, and such. For the latex particle, those generally used in an agglutination reaction may be used. Polystyrene, polyvinyl toluene, and polymethacrylate latex particles are known. A preferred carrier particle is a polystyrene latex particle. It is possible to use latex particles that have surfaces onto which a functional group has been introduced through copolymerization of monomers having the functional group. Latex particles having a functional group, such as -COOH, -OH, -$NH_2$, or -$SO_3$, are known. A binding partner can be chemically linked to latex particles having a functional group.

The mean particle diameter of a carrier particle preferably ranges from, for example, 0.5 to 20 $\mu$m for a latex particle. A mean particle diameter below 0.5 $\mu$m or above 20 $\mu$m is unfavorable because pearl chain formation can hardly be achieved. The mean particle diameter of a carrier particle may be, for example, in the range of 2 to 10 $\mu$m, more preferably in the range of 1 to 10 $\mu$m, most preferably in the range of 2 to 5 $\mu$m, when it is a latex particle. Smaller carrier particles may be used if they are oval particles showing strong dielectric polarization.

In contrast to the 0.05- to 0.6-$\mu$m carrier particles used in the conventional methods of latex agglutination turbidimetry, 1-$\mu$m or larger particles can be used in the methods of the present invention. Agglutination reaction is accelerated by using the step of applying voltage pulses. As a result, agglutination reaction proceeds adequately in a short time even when larger particles are used. Larger carrier particles have the benefits described below. First, apertures with a larger diameter size can be used for particle measurement and as a result, apertures are hardly clogged. In addition, larger carrier particles can be easily distinguished from the measurement-interfering substances in body fluids. Measurement accuracy is improved as a result.

A binding partner can be linked to particle carriers by methods suitable for the material. Those skilled in the art can appropriately select a method for linking the two. For example, latex particles can physically adsorb a protein such as an antigen, an antibody, or a fragment thereof. When latex particles have a functional group on their surface, a substituent that can be covalently linked to the functional group may be linked chemically. For example, $-NH_2$ in a protein can be linked to latex having -COOH.

Carrier particles bound to a binding partner may be subjected to blocking treatment, if required. Specifically, the binding of non-specific proteins onto the surface of carrier particles can be prevented by treating the surface of carrier particles with an inactive protein. Bovine serum albumin, skimmed milk, or such can be used as an inactive protein. Furthermore, detergents or sugars may be added to the dispersion medium to improve the dispersibility of carrier particles. Alternatively, antimicrobial agents may be added to particle carriers to prevent the growth of microorganisms.

The present invention comprises the step of applying voltage pulses to a reaction solution containing an affinity substance and carrier particles. A method that aligns carrier particles in an electric field to perform an agglutination reaction is known (JP-A No. Hei 7-83928). Specifically, carrier particles can be aligned along an electric field by applying voltage pulses to a reaction solution containing an affinity substance and carrier particles.

When the principle of agglutination inhibition reaction is applied, an affinity substance and carrier particles are aligned in the presence of an agglutination reagent. The agglutination reagent can be contacted after carrier particles have been contacted with a affinity substance to be measured. Alternatively, these three components can be contacted simultaneously by adding carrier particles to a premixture containing a affinity substance to be measured and an agglutination reagent.

An alternating current component or a direct current component can be used for the voltage pulse, and these two may be combined at one's choice. An alternating voltage is preferable in that it allows reaction solutions to undergo electrolysis easily. For an alternating voltage, square waves, rectangular waves, sine waves, or such can be used. The power supply frequency for an alternating voltage can be adjusted arbitrarily depending on the ionic strength of the reaction solution (reagent). An alternating voltage is applied to provide an electric field strength of 5-50 V/mm at its peak wave value. When the electric field strength is less than 5 V/mm, carriers can hardly form pearl chains and as a result, the acceleration of agglutination reaction becomes inadequate. When the electric field strength is greater than 50 V/mm, reaction solutions readily undergo electrolysis, making it difficult to measure agglutination reactions. More preferably, voltage is applied to provide an electric field strength of 10 to 20 V/mm. The alternating current frequency is preferably in the range of 10 KHz to 10 MHz, and more preferably in the range of 50 KHz to 1 MHz.

Herein, the voltage pulse typically refers to a voltage having a wave or waveform whose amplitude undergoes transitions from a steady state to a particular level, maintains the level for a finite time, and then returns to the original state. Alternating voltage is representative of such a voltage pulse. Alternating voltage is a periodic function of time with an average voltage value of zero. Alternating voltages include sine wave, rectangular wave, square wave, and sawtooth wave voltages, which have obvious periodic amplitudes. In general, the positive electric potential and the negative electric potential in an arbitrary cycle of alternating voltage have equal areas, making the sum of the two zero. Each area is defined by the curve above or below the horizontal axis, where the electric potential difference is zero. In the present invention, voltage pulses are applied to prevent electrolysis of reaction solutions. Accordingly, when electrolysis does not take place in a reaction solution, or if the electrophoresis, when actually occurs, can be suppressed to an extent that does not substantially interfere with the reaction, voltage pulses having a non-zero sum of positive and negative electric potentials may be applied.

Herein, the square wave or rectangular wave voltage pulse refers to a power supply that comprises cycles of positive electric potential/zero electric potential difference/negative electric potential and a constant voltage for at least either the positive or negative electric potential. The time interval between a state of zero electric potential difference and the succeeding zero state in square waves or rectangular waves is referred to as pulse width. Square wave refers to voltage pulses that form a nearly tetragonal shape when its voltage changes are drafted in a graph that has voltage on the vertical axis and time on the horizontal axis. The term "tetragonal" includes squares and rectangles. In contrast, rectangular waves are voltage pulses that have a rectangular shape, which does not include squares. Thus, square waves include rectangular waves. In the present invention, a generally preferred pulse width is 50 $\mu$sec or less, for example, in the range of 0.1 to 10 $\mu$sec.

There are no limitations on the duration of zero electric potential difference in square waves or rectangular waves. In general, the electric potential difference is zero at the moment of transition between positive and negative electric potentials. However, voltage pulses that maintain zero electric potential difference for a longer period may also be used in the present invention. For example, cycles of positive/negative electric potentials having a pulse width of 0.1 to 10 $\mu$sec may comprise a condition of zero electric potential difference that lasts 0.1 to 100 $\mu$sec.

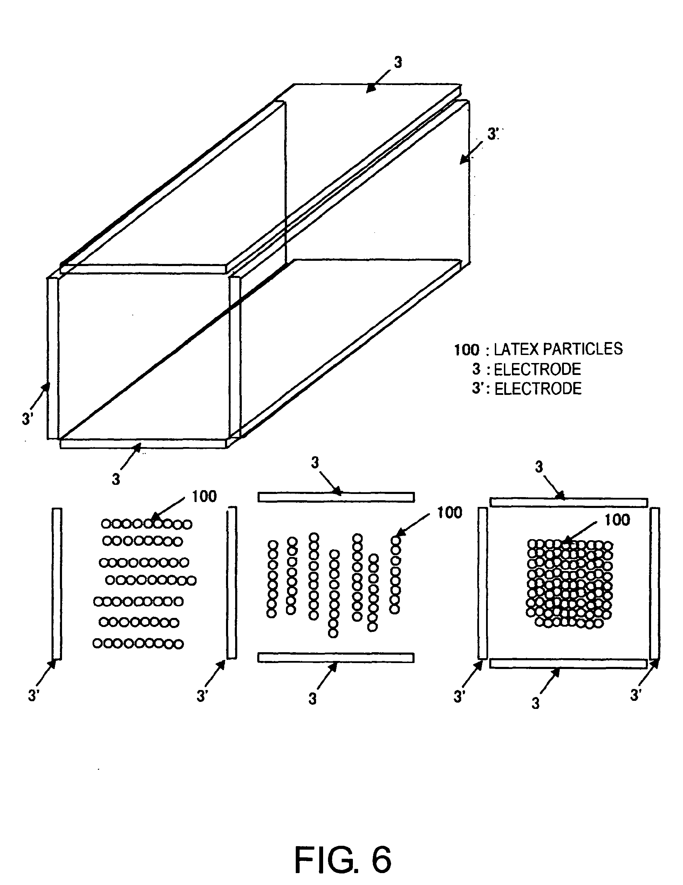

In the present invention, voltage pulses may be applied to a reaction solution from an arbitrary direction. For example, voltage pulses may also be applied in two or more different directions. Specifically, as shown in Fig. 6, voltage pulses may be applied to a reaction solution, for example, using a combination of two pairs of electrodes. Alternatively, voltage pulses may be applied to a reaction solution from a different direction by moving electrodes with respect to the reaction solution. For example, voltage pulses can be applied from an arbitrary angle by rotating electrodes. The number of electrodes moved may be one pair, or two or more pairs.

Fig. 6 shows a structure of pearl chains formed using a combination of two pairs of electrodes that are capable of providing perpendicular electric fields in the present invention. In the example shown in Fig. 6, when voltage pulses are applied in two alternating directions, carrier particles converge into a voltage pulse-crossing region (Fig. 6, bottom right). Alternatively, when application and dispersion of voltage pulses are alternately applied, pearl chains are alternately formed along the voltage pulse between electrodes 3-3 (Fig. 6, bottom central) and along the voltage pulse between electrode 3'-3' (Fig. 6, bottom left). The formation of such three-dimensional pearl chains accelerates the reaction between affinity substances.

In the present invention, two or more pairs of electrodes can be arranged arbitrarily. The length of pearl chains formed becomes shorter when the distance between electrodes is shorter. In contrast, when the distance between electrodes is greater, the applied voltage becomes greater. In fact, the structure of reaction space depends on the arrangement of electrodes. Thus, a typical distance between electrodes ranges from 0.01 mm to several tens of millimeters. In a general immunological particle agglutination reaction, electrodes may be arranged preferably by a distance range of 0.1 to 5 mm, for example, 0.5 to 3 mm.

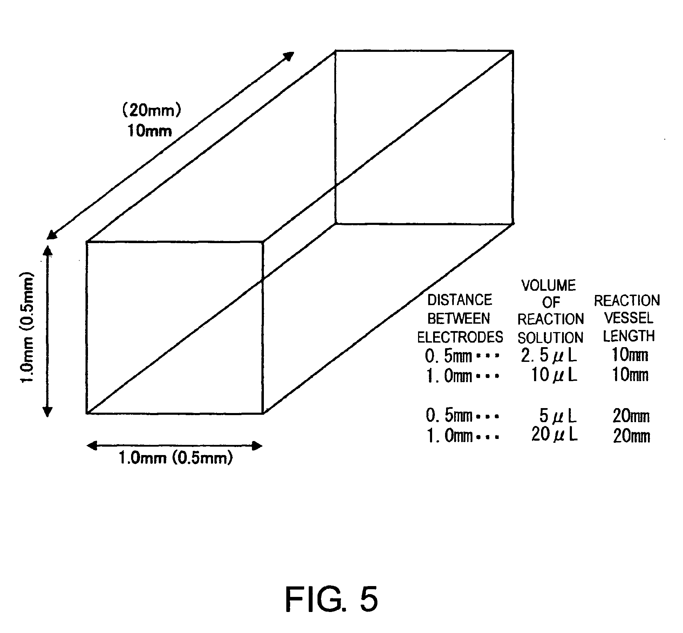

Fig. 5 shows examples of the distance between electrodes and the reaction solution volume. When electrodes are arranged at a distance range of 0.5 to 1 mm and the reaction vessel length is adjusted to 10 to 20 mm, 2.5 to 20 $\mu$l of a reaction solution, which is the standard volume of reaction solution used in an immunological agglutination reaction, can be accommodated therein. When two or more pairs of electrodes are used, it is preferable to adjust the electrode distance and height between the pairs.

A method that uses the agglutination of monoclonal antibody-sensitized carrier particles for detection of antigenic substances is known. In general, a monoclonal antibody recognizes a single epitope in an antigen. Accordingly, use of two or more types of monoclonal antibodies is generally required to agglutinate carrier particles. However, when only monoclonal antibodies are used, the number of antibodies (namely, the number of carrier particles) capable of binding to a single antigen molecule, in principle, does not exceed the number of monoclonal antibody types used. This is one of the factors that limit the detection sensitivity in analysis methods that are based on agglutination reaction of carrier particles using monoclonal antibodies. If voltage pulses can be applied in two or more directions based on the present invention, changing the direction of application increase the chance of contact between carrier particles and antigen molecules, and as a result, improvement of detection sensitivity can be expected.

The direction of voltage pulse application in known methods that use glass slides is limited. Three-dimensional reaction spaces are not available for methods that use a microscope or such for observation. However, in the present invention, the form of reaction space is not limited because agglutination rate is analyzed by gathering three-dimensional information on unagglutinated or agglutinated particles. A complex comprising two or more particles linked via the bonding between affinity substances is referred to as an "agglutinate (aggregate)". There is no limitation on the number of particles constituting an agglutinate. Two or more particles can be linked linearly or in a lattice-like form (matrix shape). An agglutinate is an agglutinate regardless of shape, as long as two or more particles are linked together. Thus, the application of voltage pulses in two or more different directions is a major advantage accomplished by the present invention.

In general, as the concentration of carrier particles in a reaction system becomes higher, pearl chain formation is enhanced and agglutination is accelerated. However, the agglutination rate of carrier particles re-dispersed in the absence of a biologically specific reactive substance (background) tends to increase as the carrier particle concentration increases. In a known method that observes agglutinated particles based on two-dimensional information (JP-A No. Hei 7-83928), the higher the carrier particle concentration, the higher the possibility that unagglutinated particles are mistaken as agglutinated particles. The particles are closer to each other as the particle concentration becomes higher, and thus it becomes difficult to distinguish particle agglutinates formed by agglutination from particles that are simply overlapping. Therefore, it is necessary to keep the particle concentration low in order to specifically distinguish agglutinates. Specifically, in the case of latex particles, the concentration of carrier particles in a reaction system, such as that disclosed in JP-A No. Hei 7-83928, is preferably in the range of 0.01 to 1% W/W, more preferably in the range of 0.025 to 0.5% W/W, most preferably in the range of 0.05 to 0.1 % W/W. However, such particle concentrations are not necessarily the optimal conditions for pearl chain formation. That is, in agglutinate-counting methods that are based on two-dimensional information, specific identification of agglutinates is done at the sacrifice of particle concentration.

In the present invention, agglutinates can be specifically identified regardless of the particle concentration because measurement is based on the three-dimensional information of agglutinated particles. Thus, the present invention can provide optimal conditions for pearl chain formation. That is, the carrier particle concentration can be decided by taking into consideration the balance between a affinity substance to be measured and its binding partner having binding activity. Specific detection of agglutinates can be achieved even if a high carrier particle concentration is selected. Usually, in the case of latex particles, the concentration of carrier particles in a reaction system in the present invention is preferably in the range of 0.01 to 5% W/W, and more preferably in the range of 0.1 to 2% W/W. This concentration range is two to ten times higher than that of two-dimensional information-based methods. The optimal carrier particle concentration can be appropriately adjusted depending on the carrier particle size, measurement sensitivity for the target affinity substance, and such.

In the present invention, salts may be added to a reaction solution to accelerate agglutination reaction. For example, a relatively high (10 mM or higher) concentration of salt may be added to accelerate agglutination reaction. However, a salt concentration of 600 mM or higher in a reaction system is unfavorable because such a higher concentration promotes electrolysis of the reaction solution. The salt concentration is more preferably in the range of 10 to 300 mM, most preferably in the range of 25 to 150 mM. When there is a possibility that a biological sample itself might contain a salt that accelerates agglutination reaction, the reagent's salt concentration may be adjusted so that the final salt concentration in a reaction solution falls within the range shown above. When direct-current voltage pulses are used, electrolysis takes place in a reaction solution even at a salt concentration of about 6 mM. Therefore, it is difficult to measure the biologically specific agglutination reaction in the presence of a salt.

Salts of the present invention can be selected from those that accelerate biologically specific agglutination reactions. Such salts include but are not limited to, for example, sodium chloride, potassium chloride, sodium nitrate, potassium nitrate, and ammonium chloride. A preferred salt of the present invention gives 100 $cm^2/(\Omega \cdot mol)$ or higher molar electric conductivity in a 10mM aqueous solution at 25°C. More specifically, such preferred salts include, for example, sodium chloride, potassium chloride, and ammonium chloride.

In the present invention, there are no limitations on the type of sample that contains an affinity substance. Specifically, it is possible to use an arbitrary sample that contains a affinity substance to be measured. For example, blood samples, samples collected from parts of the pharynx or such, saliva, sputum, urine, and feces are representative of biological samples. Other biological materials collected from a living body can also be used as samples for measuring biological substances in the present invention. Furthermore, cultures that are obtained by culturing such biological samples can be used as samples of the present invention. The biological materials can be used as samples directly, or if required, after being processed. For example, the biological materials may be used as samples after treatment of fractionation, dilution, lysis, extraction, or such.

The present invention comprises the step of measuring particles' three-dimensional information. Accordingly, for samples that contain solid components, it is preferable to preliminarily remove the solid components by elimination or dissolution. The solid components can be removed by filtration or centrifugation. However, the removal of solid components is not essential if signals of carrier particles can be clearly distinguished from signals of the solid components derived from the sample using particle size information or such.

In the present invention, samples used for the measurement may be a stock solution or an automatically diluted solution. The dilution fold may be set arbitrarily. When several types of reagents are required for a reaction, they may be added successively.

Herein, reagents that constitute a second reagent include, for example, the following reagents.

Reagents that preliminarily decompose and/or absorb substances that cause nonspecific reactions may be used in the present invention. Such reagents can be used as reagents that comprise a nonspecific reaction-suppressing agent. In combination, reagents comprising a nonspecific reaction-suppressing agent and reagents comprising carrier particles constitute the first and the second reagents. Reagents comprising a nonspecific reaction-suppressing agent may be preliminarily mixed with a sample, for example. For example, conventionally known agents that suppress nonspecific reactions may be used.

Immunoassay reveals the presence of various substances that cause nonspecific reactions in a sample. For example, globulins, such as rheumatoid factor, may interfere with the immunological reactions that make up an immunoassay. Agents that suppress nonspecific reactions may be used to prevent the globulin interference of immunoassay. For example, nonspecific effects can be absorbed by globulin-recognizing antibodies. The rheumatoid factor is a globulin derived from IgG or IgM, and can therefore be absorbed using an anti-human IgG antibody or an anti-human IgM antibody. Methods that prevent interference by decomposing causative substances of nonspecific reactions are known. Specifically, it is known that the interfering effects of globulins can be suppressed by reducing globulins to decomposition. The reduction of globulins can be achieved using dithiothreitol, 2-mercaptoethanol, or such.

Alternatively, it is possible to combine two or more types of reagents comprising carrier particles that are bound to binding partners having different binding activities. Such constitution allows different types of target affinity substances of measurement to be measured at a time. Each reagent can be added separately. Alternatively, a sample can be mixed with two or more preliminarily mixed reagents.

It is preferable to mix sample with reagents before voltage application. The two may be physically mixed using a stirrer bar. Alternatively, the two may be mixed by an electric means. Examples of electric means include a method that comprises physically displacing the positions of carrier particles by intermittently applying voltage pulses in different directions.

The present invention relates to methods for measuring affinity substances, which comprise:

(1) a step of combining carrier particles with a affinity substance to be measured and aligning the carrier particles along an electric field by applying voltage pulses, wherein the carrier particles are bound to a binding partner having an activity to bind the affinity substance to be measured;

(2) a step of counting carrier particle agglutinates formed upon binding of the affinity substance to be measured, or unagglutinated carrier particles which do not bind to the affinity substance to be measured, or both, using their three-dimensional information as an indicator; and

(3) a step of determining the level of the target substance of measurement based on the level of agglutinate formation or the level of unagglutinated carrier particles, or both.

Steps that make up the measurement methods of the present invention are specifically described below.

The mixed reaction solution is then transferred into a vessel equipped with electrodes, to which voltage pulses are applied. When an electric field is applied, dielectric polarization is induced, and carrier particles attract each other and align linearly. This phenomenon is called pearl chain formation. The linearly aligned carrier particles re-disperse immediately after the electric field is removed. However, if a biologically specific reactive substance is present during pearl chain formation, carrier particles participating in the biologically specific reaction remain as agglutinates and do not disperse even after removal of the electric field. The presence of a biologically specific reactive substance can be detected or measured by measuring the agglutinated particles that have participated in a biologically specific agglutination reaction and/or the non-participating dispersed carrier particles.

The measurement methods of the present invention comprise counting carrier particle agglutinates formed upon binding of a affinity substance to be measured, or unagglutinated carrier particles which do not bind to the affinity substance, or both, using their three dimensional information as an indicator. In the present invention, the particles can be measured after electric field is removed. Alternatively, the particles in an electric field can be measured without the electric field being removed. For example, the particles in an electric field can be counted by removing them from the electric field. Further, the process of dispersing particles can be conducted before particles are counted. Particles that have agglutinated due to nonspecific factors can be dispersed in the dispersion process before counting. As a result, improvement of measurement accuracy can be expected. Particles can also be dispersed by stirring or diluting a reaction solution.

In the present invention, the particles are counted using their three-dimensional information as an indicator. Herein, the phrase "to count using three-dimensional information as an indicator" means that the three-dimensional information of particles and/or agglutinates is measured and the particles and/or agglutinates are counted based on the result. In known methods that analyze microscopic images, the level of agglutination is determined based on two-dimensional information. Therefore, the present invention which uses three-dimensional information as an indicator is clearly different from the known methods.

There is no limitation on the method of measuring three-dimensional information. Herein, "counting" refers to determining the number of particles and/or agglutinates. The number of particles and/or agglutinates can be determined by simple counting. Alternatively, agglutinated particles and unagglutinated particles can be counted separately. Furthermore, in measuring agglutinated particles, the number of agglutinates may be determined for each number of agglutinated particles. There are known methods for counting particles using three-dimensional information as an indicator.

Measurement methods that are based on physical principles can be advantageously applied as the particle-counting methods in the present invention. Herein, "physical measurement methods" refers to measurement methods that enable the evaluation of inherent physical information of particles or agglutinates. In other words, the inherent physical information of particles or agglutinates is a result of true measurement. On the other hand, methods that analyze two-dimensional information obtained from graphic information also detect non-agglutinated overlapping particles as agglutinates. Such detection results are not considered inherent physical information of particles.

The use of a flow system is advantageous when the particles or agglutinates are measured physically. A flow system is a system which is capable of analyzing physical information of particles that pass through a minute flow cell. Physical measurements can be achieved conveniently by using a flow system. Specifically, physical measurements in the present invention comprise the step of counting by using a flow system to measure the three-dimensional information of particles and/or agglutinates. Methods that use three-dimensional information as an indicator to physically count particles include, for example, the Coulter principle and laser diffraction/scattering methods.

The Coulter principle (USPA 2656508 in 1953) is an analysis method for determining the volume of a particle based on the change of electric resistance resulted from passing of the particle through an aperture (small hole), which has electrodes on both sides. When a minute electric current is allowed to pass through an electrolytic solution between two electrodes, particles that are suspended in the electrolytic solution are aspirated, passed through an aperture, and then replaced by an equivalent volume of electrolytic solution. As a result, the electric resistance between electrodes is altered. The particle number and size (volume) can be determined by measuring this change. The electrostatic capacity method is available as a method for measuring volume; however, most of the methods that are in practical use are electric resistance methods.

The aperture size can be appropriately adjusted to accommodate the subject particle of analysis. When agglutination of carrier particles such as those used in general immunological particle agglutination reactions is detected, the aperture size is typically in the range of 30 to 1000 μm, and preferably in the range of 50 to 200 μm.

It is advantageous to have an aperture size that is several to several hundred times greater, for example, several to a hundred times greater, preferably 5 to 50 times greater than the mean particle diameter of carrier particles. In this case, highly accurate and sensitive measurements can be realized by detection of signals proportional to the volume. The sensitivity is higher when the aperture size-to-particle diameter ratio is small. However, when the ratio is too small, particles tend to clog up the aperture; when the ratio is too large, sensitivity of particle detection decreases; both cases are unfavorable.

More specifically, when the carrier particles to be counted have a particle diameter of, for example, 1 to 5 μm, particularly 2 to 3 μm, the aperture size may be selected from a range of 30 to 100 μm, preferably 50 to 80 μm, for example, 65 to 75 μm. Carrier particles that have a size of 2 to 3 μm are particularly preferred in the methods for measuring affinity substances by the present invention.

Specifically, the present invention provides methods for measuring affinity substances, which comprise:

(1) a step of combining carrier particles having a mean particle diameter of 2 to 3 μm with a affinity substance to be measured and applying voltage pulses, wherein the carrier particles are bound to a binding partner having an activity to bind the affinity substance to be measured; or (1') a step of combining carrier particles having a mean particle diameter of 2 to 3 μm with a affinity substance to be measured and an agglutination reagent component, and applying voltage pulses, wherein the carrier particles are bound to a binding partner having an activity to bind the affinity substance to be measured, and wherein the affinity substance to be measured inhibits agglutination of the carrier particles by the agglutination reagent;

(2) a step of counting carrier particle agglutinates formed upon binding of the affinity substance to be measured, or unagglutinated carrier particles which do not bind to the affinity substance to be measured, or both, using their three-dimensional information as an indicator after step (1), wherein an aperture of size 50 to 80 μm according to the Coulter principle is used; or

(2') a step of counting carrier particle agglutinates formed upon binding of the agglutination reagent, or carrier particles of which agglutination is inhibited through binding of the affinity substance to be measured, using their three-dimensional information as an indicator after step (1'), wherein an aperture of size 50 to 80 μm according to the Coulter principle is used; and

(3) a step of determining the level of the target substance of measurement based on either or both of the level of agglutinate formation and the level of unagglutinated carrier particles after step (2) or (2').

In general, the smaller the aperture size, the more accurately unagglutinated particles can be counted. Conversely, greater aperture size reduces the chance of an aperture being clogged with agglutinated particles. Aperture clogging decreases analysis efficiency, which can be improved by reducing the clogging frequency. For example, if agglutinated particles are predicted to be formed in great numbers, aperture clogging can be prevented by setting the aperture size to be slightly larger. Alternatively, a similar effect can be expected by using carrier particles with a small particle diameter. Further, the proportion of agglutinated particles may be reduced by diluting the sample to thereby prevent aperture clogging. In general, appropriate conditions may be selected for each case depending on the expected detection sensitivity, the predicted concentration of target substance to be detected, and the device configuration (aperture size, in particular).

The proportion of agglutinated particles can be determined by counting agglutinated particles by the procedure described above. The "proportion of agglutinated particles" refers to the proportion of agglutinated particles among the total particles counted. The proportion of agglutinated particles is also referred to as "agglutination rate (aggregation rate)". Furthermore, agglutination rate is determined for standard samples with known analyte concentrations, and the relation between the two is plotted on a graph to produce a standard curve. The concentration of a affinity substance to be measured in a sample can be revealed by checking the sample's agglutination rate against the graph.

Alternatively, the above-mentioned standard curve can also be expressed as a regression equation. Once a regression equation is obtained, the concentration of a affinity substance to be measured can be calculated by substituting the agglutination rate into the regression equation.

On the other hand, laser diffraction/scattering methods are used to count particles and measure their mean diameter by detecting fluctuations generated from laser irradiation of particles. In either case, for the purpose of improving measurement accuracy, it is preferable to dilute reaction particles, apply sonication, and/or use a sheath flow system, and such to prevent false measurements of particles.

Methods for measuring particle volume also include the methods described below.

Centrifugal sedimentation method: a method for determining particle diameter distribution by the Stokes equation, which represents the relation between particle sedimentation rate in a solution and particle diameter. Photocentrifugal sedimentation methods use a phenomenon based on Stokes' law: larger particles sediment faster than smaller ones when they have the same specific gravity. The particle concentration is analyzed as the change in turbidity from light transmission. The particle size distribution can be determined by the procedure described above.

Capillary system: Poiseuille flow is generated in a capillary when the viscous fluid that flows through the capillary has a low Reynolds number. Since this flow is faster at the center of the capillary and slower near the capillary wall, large particles travel in fluxes that are faster on average and smaller particles travel in fluxes that are slower on average. Briefly, particles traveling through a capillary of given length are size-separated and detected according to the differences of their moving velocities.

Three-dimensional image analysis: Three-dimensional particle information can be obtained by analyzing graphic information of two or more images taken from different angles. Alternatively, three-dimensional particle information can be obtained by scanning graphic information along the z axis in the xy plane.

In the measurement methods of the present invention, agglutinated (or unagglutinated) carrier particles are counted using three-dimensional information as an indicator. The target affinity substance of the measurement is measured qualitatively or quantitatively based on the counting results. In such qualitative measurements, the presence of a affinity substance to be measured is indicated by the presence of agglutinated particles. Alternatively, detection of agglutination inhibition in an agglutination inhibition reaction proves the presence of the target of measurement.

Alternatively, in such quantitative measurements, the level of agglutination can be correlated with the amount of affinity substance to be measured. More specifically, samples containing a known concentration of affinity substance are measured preliminarily using the measurement methods of the present invention to unravel the relation between the amount of affinity substance and the result of agglutinated particle detection based on three-dimensional information. Then, samples are measured by the same measurement procedure. The amount of affinity substance can be determined from the result of agglutinated particle detection based on volume. In the case of an agglutination inhibition reaction, quantitative measurements can also be achieved by the same procedure described above.

In methods for counting particles and/or agglutinates, formulae such as [number of particles that form agglutinates of two or more particles]/[total number of particles], or [number of single particles]/[total number of particles], can be selected as means for counting a specific number of particles according to the purpose. The total number of particles may be determined as the total number of particles measured within a fixed time period of measurement, or in a literal sense, the total number of particles in a reaction solution when the entire reaction solution is the target of analysis. When the total volume of a reaction solution is known, the total number of particles in a reaction solution can be estimated by counting a portion of the reaction solution.

Alternatively, the affinity substance can be detected or measured based on the number of particles and/or agglutinates detected during a certain period of time by an electric resistance method, laser diffraction/scattering method, or such. That is, the number of particles counted decreases with time because single particles agglutinate to form agglutinates in agglutination reactions. Alternatively, it is possible to use the time required for counting a specific number of particles and/or agglutinates as an indicator. When such counting methods are used in the present invention, the relation between the number of particles and/or agglutinates and the amount of affinity substance can be expressed in a regression equation.

For particles that have been sensitized with an antibody, the proportion of agglutinates comprising two or more particles increases depending on the antigen concentration. In this case, the agglutination rate represented by [number of particles forming agglutinates consisted of two or more particles]/[total number of particles] converges to 1.00 (100%).

When compared with methods that analyze two-dimensional graphic data, methods that measure three-dimensional particle information, whether it be the Coulter principle or a laser diffraction/scattering method, allow high-accuracy analyses even with a simple device configuration. As described above, the volume of reaction solution is restricted in analyses of two-dimensional graphic data. In contrast, there are no limitations on the reaction solution volume in methods that measure three-dimensional information using flow-based analytical techniques. In addition, there are no limitations on the physical geometry of reaction space. These reasons attribute to a simpler device configuration. The fact that the reaction solution volume can be set freely further contributes to the reproducibility and detection sensitivity.

The present invention relates to methods for measuring affinity substances, which comprise:

(1) a step of combining a affinity substance to be measured with an agglutination reagent and carrier particles that are bound to a binding partner having an activity to bind the affinity substance to be measured, and aligning the carrier particles along an electric field by applying voltage pulses, wherein the carrier particles are agglutinated by the agglutination reagent and the agglutination is inhibited by the affinity substance to be measured;
(2) a step of counting carrier particle agglutinates formed upon binding of the agglutination reagent or carrier particles whose agglutination is inhibited by the binding of the affinity substance to be measured, or both, using their three-dimensional information as an indicator; and
(3) a step of determining the level of the target substance of measurement based on either or both of the level of agglutinate formation and the level of unagglutinated carrier particles.

The following describes principles for the immunological particle agglutination reaction based on agglutination inhibition reactions that use agglutination reagents. The present invention can be applied to immunological particle agglutination reactions by using the steps described above. Steps consisting of applying voltage pulses and analyzing levels of agglutinate formation or levels of unagglutinated carrier particles can be achieved by the specifically described methods above.

When the present invention is implemented based on the principle of agglutination inhibition reaction, it is preferable to select conditions that allow a larger number of agglutinates comprising two or more particles to be formed. Alternatively, methods for evaluating the level of agglutination using [number of single particles]/[total number of particles] as an indicator are preferred. When the principle of agglutination inhibition reaction is applied, use of the above formula can be expected to provide a higher sensitivity than analyses based on the [number of particles forming agglutinates consisted of two or more particles]/[total number of particles] formula.

The present invention also provides devices for carrying out the measurement methods described above. Specifically, the present invention relates to devices for measuring affinity substances, which comprise the following tools:

(a) a space that contains carrier particles and a affinity substance to be measured, wherein the carrier particles are bound to a binding partner having an activity to bind to the affinity substance to be measured;
(b) electrodes for applying voltage pulses to the carrier particles in the above-mentioned space; and
(c) a device for counting either or both of the agglutinates formed through agglutination of carrier particles and the unagglutinated carrier particles in the above-mentioned space using their three-dimensional information as an indicator.

In the present invention, any space appropriate for containing a reaction solution may be used as (a) a space to contain carrier particles and a affinity substance to be measured, wherein the carrier particles are bound to a binding partner having an activity to bind to the affinity substance to be measured. A low-capacity space is advantageous for carrying out reactions with trace amounts of sample. For example, a space of 1 $\mu$l to 10 ml, preferably 10 to 500 $\mu$l, can be used. If required, the space may contain a device for supplying samples and reagents, or a device for measuring carrier particles as described below.

The electrodes (b) used in the present invention to apply voltage pulses to carrier particles in the above-mentioned space are described below. The electrodes used for aligning carrier particles in an electric field are also used, for example, in the prior art documents indicated above. Such known electrodes may be used in the present invention. The devices of the present invention can be equipped with a power source(s) for applying voltages to the electrodes.

In the devices of the present invention, the electrodes used for applying voltage pulses are constructed from at least a pair (two) of electrodes. The devices may be equipped with three or more electrodes for applying voltage pulses in two or more different directions. For example, three electrodes: A, B, and C, are arranged, and voltage pulses may be applied in three directions: between A and B, between B and C, and between A and C. Alternatively, when two pairs (four) of electrodes are arranged, perpendicular voltage pulses may be applied (Fig. 6).

Furthermore, the devices may be equipped with a mechanism to move the electrodes so that voltage pulses can be applied in different directions. For example, voltage pulses can be applied in two or more different directions by rotating the electrodes in a reaction solution. When voltage pulses are applied in different directions, any angles can be used.

Furthermore, the devices of the present invention comprise a device (c) for counting either or both of the agglutinates formed through agglutination of carrier particles and the unagglutinated carrier particles in the above-mentioned space using their three-dimensional information as an indicator. The above-described space may be equipped with the counting device. Alternatively, counting can be carried out after a reaction solution is taken from the above-described space and introduced into the counting device.

Measurement devices that apply the Coulter principle or a laser diffraction/scattering method can be used as means for counting agglutinated or unagglutinated carrier particles using three-dimensional information as an indicator. When the Coulter principle is used, for example, a reaction solution is transferred from the above-mentioned space to an aperture equipped with Coulter-principle electrodes to carry out the required analyses. The aperture size can be adjusted appropriately based on the criteria described above. It is possible to employ a structural body to switch between two or more apertures of different sizes, and use them according to the diameter of particles used as the reagent or the predicted proportion of agglutinated particles. The devices of the present invention may be equipped, for example, with a structural body that switches the flow path in order to transfer the reaction solution to multiple apertures. Furthermore, structural bodies that automatically select a flow path according to the reagent type, the predicted proportion of agglutinated particles, or such may be used in combination. Alternatively, the devices of the present invention may be equipped with a structural body that automatically adjusts the detection sensitivity according to the change in aperture size. The structural body for adjusting detection sensitivity includes, for example, those that analyze using a slightly larger aperture size first and switching to a smaller aperture when the proportion of agglutinated particles is predicted to be small. When a laser diffraction/scattering method is used, the analysis may be carried out by introducing the reaction solution into an optical cell for analysis by the same procedure described above.

In the present invention, three-dimensional information of the carrier particles that form pearl chains in an electric field may be obtained after being re-dispersed, if necessary. The device of the present invention may be equipped with a structural body for re-dispersing carrier particles. The carrier particles can be re-dispersed through dilution or sonication.

The above (a) to (c) elements which constitute the devices of the present invention may be placed in a single continuous flow path. Alternatively, the measurement methods of the present invention can be carried out by constructing each element as a discontinuous space and allowing a reaction solution to travel between the elements.

The devices of the present invention may be used in combination with an additional structural body for carrying out the measurement methods described above. Examples of an additional structural body that can be combined with the devices of the present invention are listed below.

Structural body for sorting samples

Structural body for diluting samples

Structural body for recording measurement results

Structural body for displaying measurement results

Structural body for printing measurement results

All prior art documents cited herein are incorporated by reference.

Brief Description of the Drawings

Fig. 1 (A) is a diagram showing the configuration of a device of the present invention.

Fig. 1 (B) is a sectional view showing a pulse-application vessel constituting a device of the present invention.

The symbols in this diagram represent the following elements.

1: Dispensing and mixing device

2: Reaction vessel

3: Electrodes (means for applying pulses)

4: Dilution device

5: Device for measuring particle size distribution

Fig. 2 is a diagram demonstrating the measurement principle for the method of measuring affinity substances according to the present invention.

The symbols in this diagram represent the following elements.

100: Latex particle

101: Antibody

102: Antigen

3: Electrode

104: Reaction vessel

Fig. 3 is a diagram showing the specific configuration of a device used to conduct the method for measuring affinity substances according to the present invention.

The symbols in this diagram represent the following elements, respectively.

1: Dispensing and mixing unit

2: Pulse application unit (3a and 3b)

3a: Electrodes (application of pulses)

3b: Pulse power source unit

4: Dilution unit

5: Unit for measuring particle size distribution

6: Aperture

7: Sheath flow

8: Electrodes (Coulter counter)

9: Pump

10: Waste fluid

11: Sheath liquid

12: Dilution solution (washing solution)
13: Computer unit

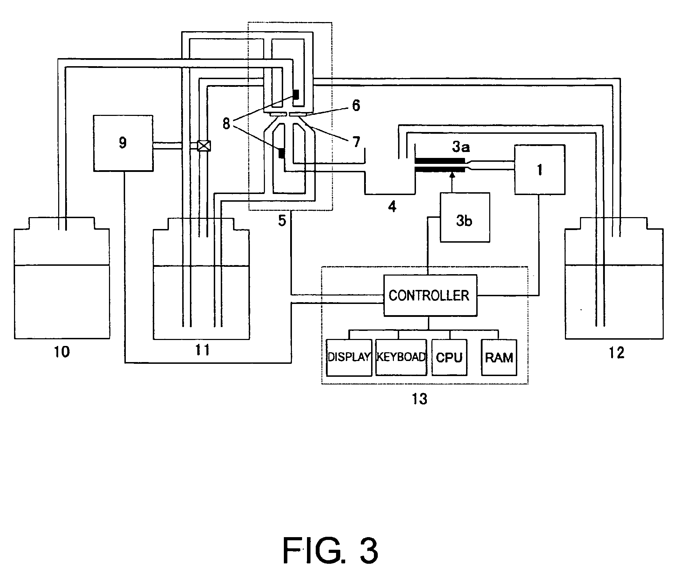

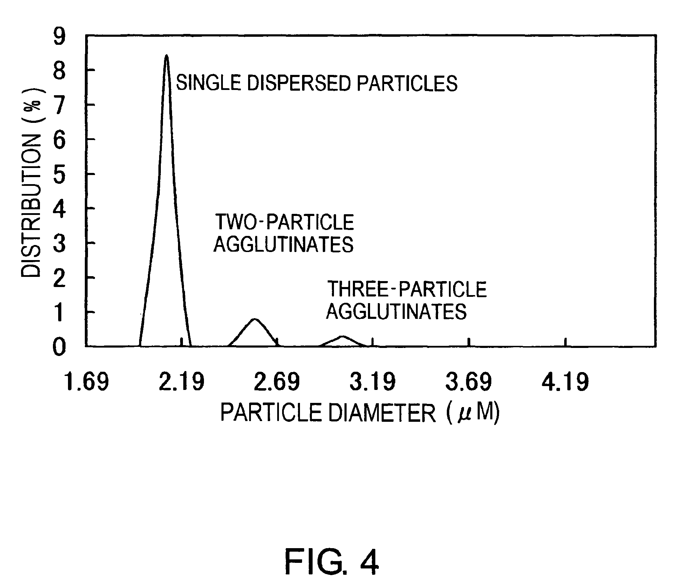

Fig. 4 is a graph showing measurement results of particle size distribution obtained by a measurement method of the present invention using a measurement device that has Fig. 3 configuration. In this diagram, the vertical axis indicates particle size distribution (%), and the horizontal axis indicates particle diameter ($\mu$m).

Fig. 5 is a diagram showing an example of the electrode arrangement and the volume of reaction space when two or more pairs of electrodes are arranged.

Fig. 6 is a diagram showing an example of the pearl chain structure formed by applying voltages to the electrodes arranged in the same way as in Fig. 5.

The symbols in this diagram represent the following elements.

3: Electrodes (first pair of electrodes)
3': Electrodes (second pair of electrodes)
100: Latex particles

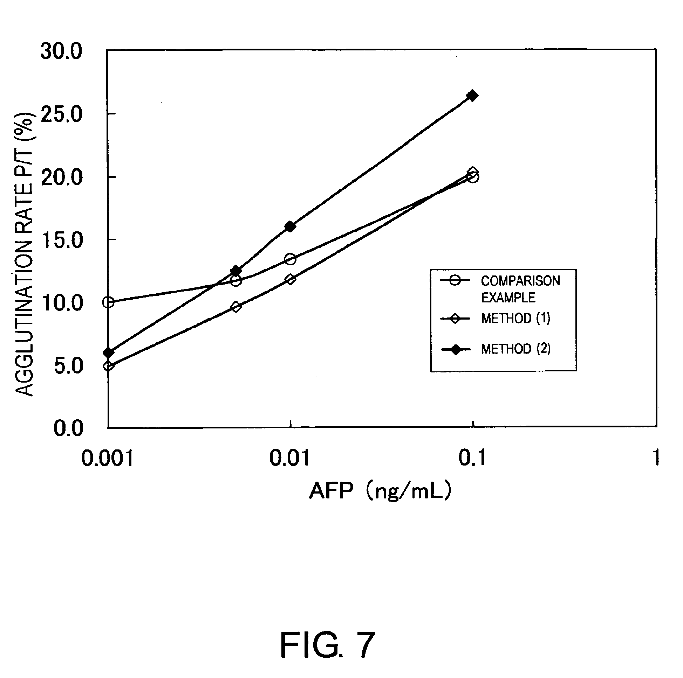

Fig. 7 is a graph showing results of comparison between the measurement methods of the present invention, which are based on three-dimensional information, and a known method that observes two-dimensional information. In this diagram, the vertical axis indicates agglutination rate (P/T%), and the horizontal axis indicates AFP concentration (ng/ml).

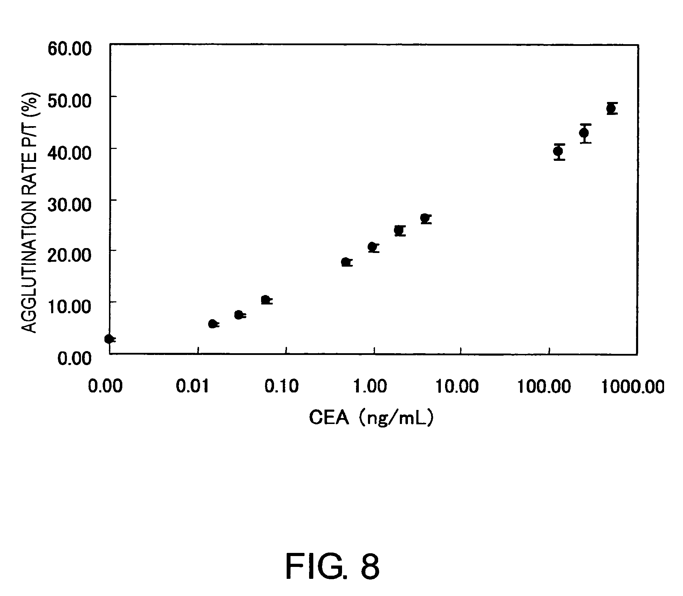

Fig. 8 is a graph showing measurement results obtained by the method of the present invention based on pearl chain formation reaction and three-dimensional information. In this diagram, the vertical axis indicates agglutination rate (P/T%), and the horizontal axis indicates CEA concentration (ng/ml).

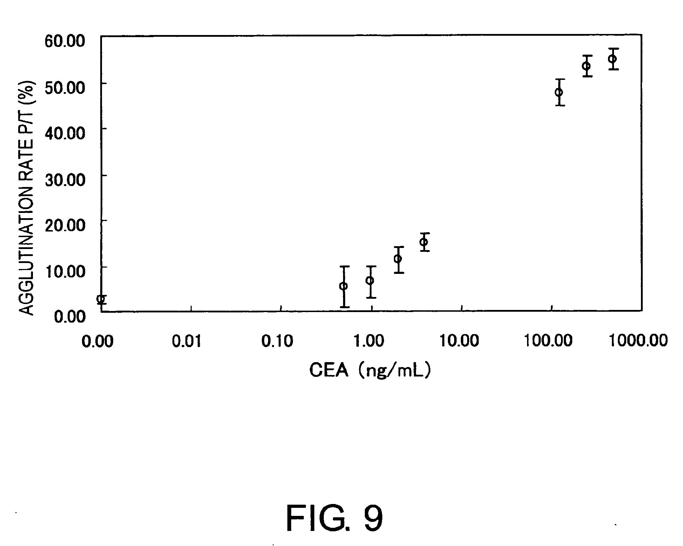

Fig. 9 is a graph showing measurement results obtained using a known method based on a 20 minute incubation reaction at 37°C and three-dimensional information. In this graph, the vertical axis indicates agglutination rate (P/T%), and the horizontal axis indicates CEA concentration (ng/ml).

Best Mode for Carrying out the Invention

The present invention is illustrated in detail below with reference to the Drawings, but is not to be construed as being limited thereto.

1. Example of measurement using a device of the present invention:

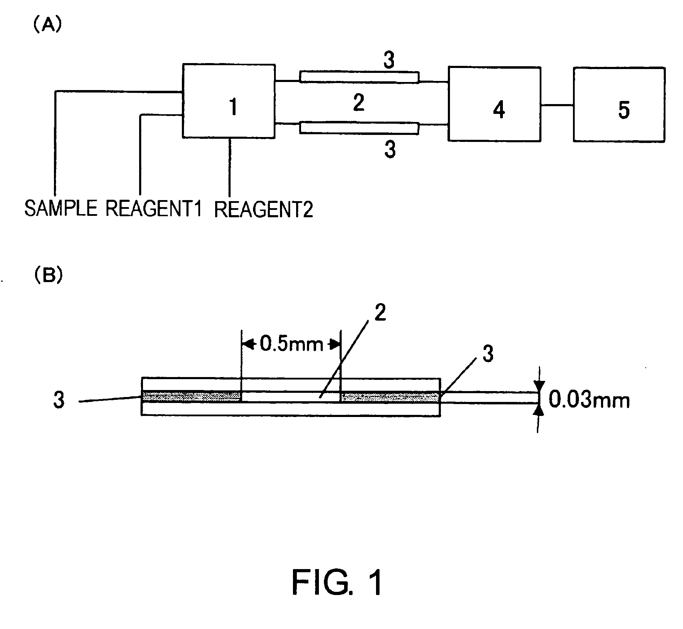

Fig. 1 (A) is a diagram showing the configuration of a device of the present invention.

This device comprises a dispensing and mixing chamber for preparation of a reaction solution by dispensing and mixing a sample with Reagent 1 (buffer), and further dispensing and mixing Reagent 2 (latex reagent) with the resulting mixture. The reaction solution is transferred to pulse-application vessel 2. Voltage pulses are applied via electrodes 3 for several seconds to several tens of seconds to achieve pearl chain formation. The reaction solution is diluted (dilution chamber 4) after pearl chain formation. Then, particle size distribution is measured.

Fig. 1 (B) is a diagram showing a sectional view of the pulse-application chamber. The distance between electrodes is 0.5 mm; electrode thickness is 0.03 mm; and electrode length is 20 mm.

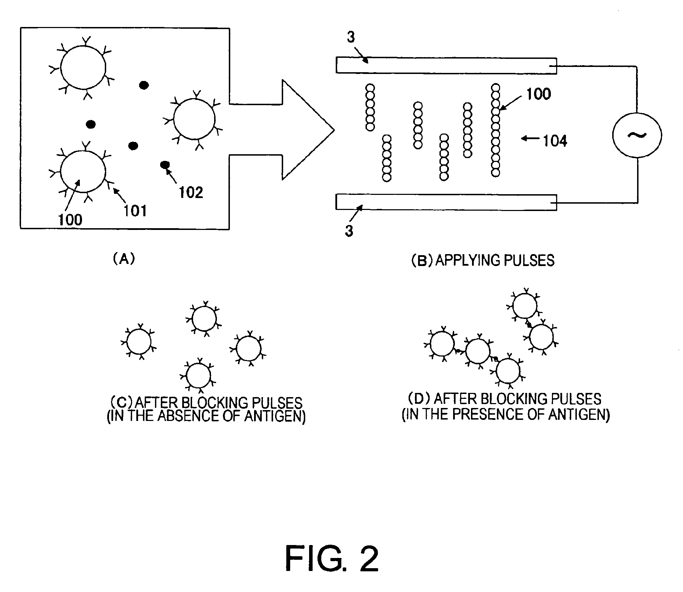

Fig. 2 is a diagram showing the measurement principle for the biologically specific reaction using the measurement device shown in Fig. 1. A reaction solution is prepared by combining a latex reagent that comprises latex particles 100 (diameter: 2 $\mu$m) bound to antibody 101, which is the target substance of measurement, and buffer-diluted sample 102. Latex particles can be linked to an antibody or antigen via hydrophobic or chemical bonds using known techniques. The sample to be used includes body fluids, such as blood and urine, extracts from air, soil, river or such.

A specific example of the device is described with reference to Fig. 3. 1 $\mu$l of serum sample and 10 $\mu$l of glycine buffer are aliquoted using dispensing and mixing unit 1. Then, a 5 $\mu$l aliquot of anti-AFP antibody-sensitized latex reagent is combined with 3 $\mu$m latex particles. The reaction solution is transferred to the reaction vessel equipped with electrode 3a, and an alternating voltage (100 KHz; 20 V/mm) is applied using pulse power source 3b for 30 seconds to achieve the pearl chain formation of latex particles. Then, the reaction solution is transferred to the dilution chamber, and diluted by adding 1 ml of dilution solution 12. Then, the dilution chamber is subjected to ultrasonic vibration for 1 second to completely re-disperse the particles that did not participate in the antigen-antibody reaction. The reaction solution is transferred to particle size distribution measurement unit 5 and passed from sheath flow system 7 through to aperture 6, at which the signal is detected by electrodes 8. The particle size distribution of latex particles is determined based on the signal. Particle size distribution obtained by the procedure described above is shown in Fig. 4. The agglutination rate (AR) of latex particles is calculated according to the equation shown below. The AFP concentration can be determined from a standard curve prepared by preliminary measurement of AFP standard samples using the same procedure described above.

**Formula 1: AR = (number of particles forming agglutinates consisted of two or more particles)/(total number of particles) x 100 (%)**

The measurement is achieved under the automatic regulation of each unit by computer unit 13 and the result is shown on the display.

2. Comparison between the measurement method (three-dimensional information) of the present invention and a known measurement method (two-dimensional information):

Methods for preparing anti-human AFP antibody-sensitized latex reagents

1.0% latex (3 $\mu$m in diameter; Polysciences Inc.) suspended in glycine buffer was added to 1.0 ml of a glycine buffer containing 0.07 mg/ml anti-human AFP antibody (IgG). After the resulting mixture was stirred at room temperature for 2 hours, the suspension of sensitized latex was centrifuged at 10000 rpm for 10 minutes and the resulting supernatant was discarded. The precipitate was suspended in a glycine buffer containing 1.0% bovine serum albumin. After 1 hour of incubation at 37°C, the suspension was again centrifuged (10000 rpm, 10 minutes) and the resulting supernatant was discarded. The precipitate was suspended in a glycine buffer containing 0.2% bovine serum albumin, 10% sucrose, 50mM NaCl, and 0.09% $NaN_3$. The anti-human AFP antibody-sensitized latex reagent was thus prepared. The following three types of measurement methods were carried out using the reagent.

Method (1)

Pearl chain formation was achieved using the anti-human AFP antibody-sensitized latex reagent and AFP standard samples (calibrator) by applying an ac voltage (100 KHz, 12 V/mm, square wave) to the mixture for 40 seconds. Immediately, the power was turned off, and the latex particles were counted with a Coulter counter. The agglutination rate was determined from the ratio of the number of particles forming agglutinates consisted of two or more particles (P) to the total number of particles (T).

Method (2)

Pearl chain formation was achieved using the anti-human AFP antibody-sensitized latex reagent and AFP standard samples (calibrator) by applying an ac voltage (100 KHz, 12 V/mm, square wave) to the mixture for 40 seconds. Immediately, the power was turned off, and the mixture was allowed to stand for 20 seconds to re-disperse the latex particles that did not participate in the specific agglutination reaction. After two repetitions of the steps of pearl chain formation and re-dispersion, latex particles were counted with a Coulter counter by the same procedure used in Method (1) to determine the agglutination rate.

Comparison example

Pearl chain formation was achieved using the anti-human AFP antibody-sensitized latex reagent and AFP standard samples (calibrator) by applying an ac voltage (100 KHz, 12 V/mm, square wave) to the mixture for 40 seconds. Immediately, the power was turned off, and the mixture was allowed to stand for 40 seconds to re-disperse the latex particles that did not participate in the specific agglutination reaction. The reaction solution was observed under a microscope. The resulting microscopic images were entered into a computer via a CCD camera, and analyzed as two-dimensional images. The agglutination rate was determined from the ratio of the number of particles forming agglutinates consisted of two or more particles (P) to the total number of particles (T).

Results

The measurement results are shown in Table 1 and Fig. 7. The agglutination rate converged to about 10% by the known measurement method which uses two-dimensional image analysis. In particular, there was little change in the agglutination rate at lower concentrations (0.001 to 0.01 ng/ml). It was speculated that unagglutinated particles were miscounted as agglutinated particles due to particle overlapping in the lower concentration range, and this had a major impact on the error. In contrast, in the three-dimensional analysis using the electric resistance method of the present invention, the agglutination rate was observed to decrease almost linearly even in the low concentration range. The result showed that the sensitivity was improved about 10 times better than the two-dimensional analysis method. Specifically, in the method of the present invention, the agglutination rate was observed to change linearly over the antigen (AFP) concentration range of 0.001 to 1000 ng/ml. The sensitivity was further improved, particularly in the concentration range of 0.1 to 100 ng/ml, through repetitions of pearl chain formation.

[Table 1]

| AFP (ng/ml) | | Agglutination rate (%) | |
|---|---|---|---|
| | Comparison example | Method (1) | Method (2) |
| 0.001 | 0.0 | 4.9 | 6.0 |
| 0.005 | 11.7 | 9.6 | 12.5 |
| 0.01 | 13.4 | 11.8 | 16.0 |
| 0.1 | 19.9 | 20.3 | 26.4 |
| 1 | 30.0 | 30.2 | 38.0 |
| 10 | 44.1 | 43.8 | 50.4 |
| 100 | 58.3 | 57.8 | 62.4 |
| 1000 | 73.6 | 72.6 | 74.8 |

[Example 2] Relation between particle measurement and aperture diameter

(1) Preparation of anti-CEA antibody-sensitized latex reagents (Reagent 2)

To 1 ml of glycine buffer (50 mM glycine, 50 mM sodium chloride, and 0.09% NaN3; hereinafter abbreviated as "GBS") 1.0 mL containing 0.1 mg of an anti-human CEA antibody (Dako), 1 ml GBS suspension of 1.0% 2-$\mu$m latex (Polysciences Inc.) was added. After 2 hours of incubation at 37°C, the suspension of sensitized latex was centrifuged (at 10000 rpm for 15 minutes), and the resulting supernatant was discarded. The precipitate was suspended in GBS containing 2% bovine serum albumin. After 1 hour of incubation at 37°C, the suspension was again centrifuged (10000 rpm, 10 minutes), and the resulting supernatant was discarded. The precipitate was suspended in GBS (pH 8.2) containing 0.2% bovine serum albumin, 10% sucrose, and 5% choline chloride to prepare an anti-human CEA antibody-sensitized latex reagent (latex concentration was 1% W/V; 2-$\mu$m reagent). A 3-$\mu$m reagent was prepared using 3-$\mu$m latex (Polysciences Inc.) by the same procedure described above.

(2) Measurement device

A Coulter counter (Beckman Coulter, Inc.) was used in combination with four types of apertures (20, 50, 70, and 100 $\mu$m in diameter).

(3) Measurement method

After 0.5 $\mu$l of a normal serum was combined with 0.5 $\mu$l of Reagent 2, the mixture was aliquoted into the electrode-equipped vessel shown in Fig. 1 (B). An ac voltage (frequency of 100 KHz, 12 V, square wave) was applied to the vessel for 60 seconds to achieve pearl chain formation. Immediately, the power was turned off, and 20 ml of physiological saline was added and the combined solution was mixed by inversion. The latex particles in the mixed reaction solution were counted with a Coulter counter.

(4) Results

The results are shown in Table 2.

[Table 2]

| | Aperture (Ap) diameter | | | |
|---|---|---|---|---|
| | 20 $\mu$m | 50 $\mu$m | 70 $\mu$m | 100 $\mu$m |
| Clogging tendency (%) | 50% or higher | 15% | 2% | 2% or lower |
| Measurement of 2-$\mu$m reagent | A; Measurable | A; Measurable | B; Measurable | C; Measurable |
| Aperture diameter /particle diameter | 10 times | 25 times | 35 times | 50 times |
| Measurement of 3-$\mu$m reagent | B; Measurable | A; Measurable | A; Measurable | B; Measurable |
| Aperture diameter /particle diameter | 6.7 times | 16.7 times | 23.3 times | 33.3 times |

In Table 2, "clogging tendency (%)" refers to the frequency of aperture clogging by carrier particles during counting, where apertures of different diameter size are used. For example, when an aperture of 20 $\mu$m diameter was used, the clogging tendency (%) was 50% or higher. This means aperture clogging by carrier particles affects the result in at least one of two measurements in counting latex particles in a reaction solution. The number of measurements is the sum of 2- and 3-$\mu$m reagents. Specifically, "clogging tendency (%)" refers to the clogging tendency of aperture.

The expression "A/B/C; Measurable, or Unmeasurable" shows the result of comparing the evaluated measurement accuracy of counting using apertures of different diameter size. The evaluation criteria are described below. SN ratio is represented by N (noise)/S (signal), to be specific, [the number of unagglutinated particles (single particles) counted as agglutinated particles by error] /[the true number of unagglutinated particles (single particles)]. In the presence of reagents having a mean particle diameter of 2 or 3 $\mu$m, unagglutinated particles (single particles) in a blank sample (the AFP concentration was below the detection limit) were measured most precisely and accurately when the aperture diameter was 20 $\mu$m. Based on the results described above, the number of unagglutinated particles (single particles) counted using an aperture size of 20 $\mu$m diameter is defined as "the true number of unagglutinated particles (single particles)". Meanwhile, the difference between "the number of unagglutinated particles (single-particles) counted with an aperture diameter" and "the true number of unagglutinated particles (single particles)" is defined as "the number of unagglutinated particles (single particles) counted as agglutinated particles by error" for each aperture diameter.

A: In counting latex particles, unagglutinated particles (single particles) and particles forming agglutinates consisted of two particles can be clearly distinguished at good reproducibility. The SN ratio is 1.5 or lower.
B: The SN ratio is more than 1.5 and less than 3.
C: Unagglutinated particles (single particles) and particles forming agglutinates consisted of two particles cannot be clearly distinguished or measured, and single particles cannot be deteceted accurately. The SN ratio is more than 3 and less than 6.

Unmeasurable: The SN ratio is 6 or higher.
The measurement accuracy is shown to decrease in the order of A/B/C. When the 2-$\mu$m latex particle reagent is measured, high accuracy counting can be achieved with an aperture of 20 to 70 $\mu$m diameter (A or B). Meanwhile, the measurement accuracy is shown to be slightly lower with an aperture of 100 $\mu$m diameter (C).
When 2-$\mu$m carrier particles are used, clogging takes place frequently with an aperture diameter of 50 $\mu$m or smaller, while measurement accuracy is inadequate with an aperture diameter of 100-$\mu$m or greater. Accordingly, it is understood that an aperture having a diameter of 50 $\mu$m or greater and less than 100 $\mu$m is suitable. In particular, an aperture of 70 $\mu$m diameter is suitable. Meanwhile, when 3-$\mu$m carrier particles are measured, 50 $\mu$m to 100 $\mu$m apertures are suitable, and apertures of 70 to 100 $\mu$m diameter are particularly suitable. In other words, it is preferable to adjust the aperture diameter to be 5 to 50 times greater than the mean particle diameter of carrier particle.

[Example 3] Relation between latex size and reactivity

(1) Preparation of anti-AFP antibody-sensitized latex reagents

Anti-human AFP antibody-sensitized latex reagents were prepared by the same procedure described in Example 1. Five types of reagents were prepared using latex particles with a diameter of 2-, 3-, 4.5-, 6-, or 10-$\mu$m. Their latex particle concentrations were adjusted to 1, 1, 3, 3, and 10%, respectively.

(2) Measurement device

A Coulter counter and two types of apertures (an aperture of 70 μm diameter was used for the 2-μm latex reagent, while an aperture of 100 μm diameter was used for the other latex reagents) were used.

(3) Measurement method

After 3 μl of a sample and 3 μl of the anti-AFP antibody-sensitized latex reagent were combined, the mixture was aliquoted into the electrode-equipped vessel shown in Fig. 1 (B). An ac voltage (frequency of 100 KHz, 14 V, square wave) was applied to the vessel for 40 seconds to achieve pearl chain formation. Immediately, the power was turned off, and 20 ml of physiological saline was added and the combined solution was mixed by inversion. The latex particles in the mixed reaction solution were counted with a Coulter counter. The agglutination rate was determined from the ratio of the number of agglutinated particles consisted of two or more particles (P) to the total number of particles (T).

(4) Result

The results are shown in Table 3.

[Table 3]

| Latex particles | | Agglutination rate (P/T%) | |
|---|---|---|---|
| Particle diameter | Latex concentration in the final reaction solution | AFP 0 (ng/ml) | AFP 1000 (ng/ml) |
| 2 μm | 0.5% | 2.3 | 47.5 |
| 3 μm | 0.5% | 2.6 | 43.0 |
| 4.5 μm | 1.5% | 3.2 | 32.7 |
| 6 μm | 1.5% | 4.7 | 20.4 |
| 10 μm | 5.0% | 0.0 | 8.67 |

Table 3 shows that the agglutination rate (signal) is as great as 40% or greater when the particle diameter is 3 μm or smaller and the agglutination rate (signal) is 20% or less when the diameter is 6 μm or greater. Together with the results shown in Table 2, these results show that the latex particle diameter of the present invention is preferably in the range of 1 to 10 μm, most preferably in the range of 2 to 5 μm.

[Example 4]

(1) Preparation of an anti-CEA antibody-sensitized latex reagent (Reagent 2)

An anti-human CEA antibody-sensitized latex reagent (latex concentration of 1% W/V) was prepared by sensitizing 2-μm latex with the anti-human CEA antibody by the same procedure described in Example 2.

(2) Preparation of glycine buffer (Reagent 1)

GBS (pH 8.2) containing 0.5% bovine serum albumin and 0.6 mg/ml mixture for suppressing nonspecific reactions was prepared as Reagent 1.

(3) Measurement device

The affinity substance (antigen) was measured by using the device shown in Fig. 1(A) and the electrode-equipped vessel shown in Fig. 1(B), based on an antigen-antibody reaction.

(4) Measurement method

A CEA antigen solution was diluted with GBS containing 0.5% bovine serum albumin to adjust its concentration to 0, 0.015, 0.03, 0.06, 0.49, 0.98, 1.95, 3.9, 125, 250, and 500 ng/ml. 1 μl of each of these samples and 5 μl of Reagent 1 were combined. The resulting mixture was incubated at 45°C for 3 minutes, and then 6 μl of Reagent 2 was added thereto. After mixing, the mixture was introduced into the electrode-equipped vessel. The latex concentration was 0.5% in the final reaction solution. An ac voltage (frequency of 100 KHz, 12V, square wave) was applied using the device described above for 60 seconds at room temperature to achieve pearl chain formation. Immediately, the power was turned off, and latex particles were counted with a Coulter counter (diameter of the aperture used was 70 $\mu$m). The agglutination rate was determined from the ratio of the number of agglutinated particles consisted of two or more particles (P) to the total number of particles (T). The results are shown in Fig. 8.

Comparison example 2

Equal amounts of each sample and the reagent prepared in Example 5 were added to a test tube, and the resulting mixtures were incubated at 37°C for 20 minutes. 0.5 $\mu$l of the reaction solution was diluted with 20 ml of physiological saline. Likewise, the agglutination rate was determined using the diluted solution, by measuring the particle size distribution of latex particles with a Coulter counter by the same procedure described in Example 5. The measurement was repeated five times by the same procedure described in Example 5. The results are shown in Tables 4 and 5, and Fig. 9.

[Table 4] Pulse: 100 KHz, $\pm$12 V, square wave

|  | 0 (ng/ml ) | 0.015 | 0.03 | 0.06 | 0.49 | 0.98 | 1.95 | 3.9 | 125 | 250 | 500 |
|---|---|---|---|---|---|---|---|---|---|---|---|
| 1 | 2.66 | 5.51 | 7.42 | 10.54 | 17.68 | 21.09 | 23.84 | 26.54 | 39.23 | 43.52 | 48.22 |
| 2 | 2.56 | 5.35 | 7.51 | 10.04 | 17.76 | 20.33 | 24.07 | 26.09 | 39.78 | 42.21 | 47.54 |
| 3 | 2.53 | 5.64 | 7.32 | 10.29 | 18.00 | 20.50 | 23.90 | 26.23 | 38.67 | 42.84 | 47.39 |
| 4 | 2.85 | 5.48 | 7.31 | 10.33 | 17.47 | 20.42 | 24.35 | 25.69 | NT | NT | NT |
| 5 | 2.57 | 5.60 | 7.55 | 10.29 | 17.82 | 20.76 | 23.48 | 26.24 | NT | NT | NT |
| Ave (%) | 2.63 | 5.52 | 7.42 | 10.30 | 17.75 | 20.62 | 23.93 | 26.16 | 39.23 | 42.86 | 47.72 |
| S.D. | 0.130 | 0.113 | 0.108 | 0.178 | 0.194 | 0.308 | 0.319 | 0.309 | 0.555 | 0.655 | 0.442 |
| 2.6 S.D | 0.338 | 0.295 | 0.282 | 0.462 | 0.505 | 0.800 | 0.830 | 0.803 | 1.443 | 1.703 | 1.150 |
| C.V. % | 4.94 | 2.05 | 1.46 | 1.73 | 1.09 | 1.49 | 1.33 | 1.18 | 1.41 | 1.53 | 0.93 |

[Table 5] Control: incubation at 37°C for 20 minutes

|  | 0(ng/mL) | 0.015 | 0.03 | 0.06 | 0.49 | 0.98 | 1.95 | 3.9 | 125 | 250 | 500 |
|---|---|---|---|---|---|---|---|---|---|---|---|
| 1 | 2.55 | NT | NT | NT | 5.19 | 5.02 | 9.82 | 15.01 | 48.23 | 54.20 | 54.68 |
| 2 | 3.24 | NT | NT | NT | 6.59 | 7.78 | 10.81 | 16.03 | 47.98 | 52.87 | 53.87 |
| 3 | 2.50 | NT | NT | NT | 3.48 | 5.24 | 10.92 | 14.11 | 46.24 | 52.64 | 55.64 |
| 4 | 2.69 | NT | NT | NT | 7.42 | 7.31 | 12.08 | 15.53 | NT | NT | NT |
| 5 | 2.43 | NT | NT | NT | 3.77 | 7.44 | 12.62 | 14.69 | NT | NT | NT |
| Ave (%) | 2.68 |  |  |  | 5.29 | 6.56 | 11.25 | 15.07 | 47.48 | 53.24 | 54.73 |
| S.D. | 0.324 |  |  |  | 1.718 | 1.318 | 1.110 | 0.746 | 1.084 | 0.842 | 0.886 |
| 2.6 S.D | 0.842 |  |  |  | 4.466 | 3.426 | 2.885 | 1.939 | 2.818 | 2.190 | 2.304 |
| C.V. (%) | 12.07 |  |  |  | 32.5 | 20.1 | 9.86 | 4.95 | 2.28 | 1.58 | 1.62 |

If the value obtained by measurement in the absence of antigen is distinguishable from the value determined at a certain antigen concentration, the concentration of the antigen can be determined. The lowest value within a measurable antigen concentration range is the detection limit. In general, as long as the average - 2.6 SD value of the agglutination rate determined at a certain concentration does not overlap with the average + 2.6 SD value of the agglutination rate determined at 0 ng/ml antigen, the antigen can be detected at or above the concentration.

The detection limit of the methods of the present invention can be estimated to be 0.015 ng/ml (Fig. 8) from comparing the detection limits in Figures 8 and 9. Meanwhile, the detection sensitivity in the conventional method is 1.9 ng/ml (Fig. 9). Thus, the sensitivity of the present invention is more than 100 times higher. In addition, the reproducibility of the present invention at each antigen concentration is also superior, and the CV value is roughly in the range of 1 to 2% (the simultaneous reproducibility at an antigen concentration of 1.95 ng/ml is 1.33% CV for the present invention and 9.86% for the conventional method). Excellent linearity is seen up to an antigen concentration of 500 ng/ml. These findings show that when compared to the conventional method, measurements by the present invention, which accelerates reactions through pearl chain formation by applying voltage pulses, can be achieved in a very short time at high sensitivity with excellent reproducibility and linearity.

Industrial Applicability

The present invention provides novel methods for measuring affinity substances using agglutination of carrier particles. The measurement methods of the present invention comprise counting agglutinates of carrier particles based on three-dimensional particle information. This allows high accuracy measurements by simpler procedures, and also makes it possible to provide low-cost measuring device configurations.

Furthermore, the geometry of reaction space for agglutination reaction is not limited because in the present invention, agglutinates of carrier particles are counted based on the three-dimensional information of particles. Thus, voltage pulses can be applied in different directions using two or more pairs of electrodes. In addition, the enlargement of reaction space is expected to improve sensitivity and reproducibility. In contrast, in conventional methods that observe agglutination of carrier particles based on two-dimensional information (area), the geometry of reaction space is markedly limited because observation of particles is restricted to the limited area in focus. Although observation area can be increased by shifting the focus, only flat scanning is possible at most. As described above, when compared to known methods, the measurement methods of the present invention enable simpler measurements with high accuracy.

Furthermore, larger carrier particles can be used because the reaction is accelerated by applying voltage pulses. As a result, improvement of measurement accuracy can be expected.

**Claims**

1. A method for measuring an affinity substance, which comprises the steps of:

(1) mixing carrier particles with an affinity substance to be measured and applying a voltage pulse, wherein the carrier particles are bound to a binding partner having an activity to bind to the affinity substance; or

(1') mixing carrier particles with an agglutination reagent component and an affinity substance to be measured, and applying a voltage pulse, wherein the carrier particles are bound to a binding partner having an activity to bind to the affinity substance, and wherein the affinity substance inhibits agglutination of the carrier particles caused by the agglutination reagent;

(2) counting agglutinates of carrier particles formed upon the binding of the affinity substance to be measured, or unagglutinated carrier particles which did not bound to the affinity substance, or both, based on their three-dimensional information as an indicator after step (1); or

(2') counting agglutinates of carrier particles formed upon the binding of the agglutination reagent, or carrier particles whose agglutination was inhibited through the binding of the affinity substance to be measured, or both based on their three-dimensional information as an indicator after step (1'); and

(3) determining the level of the substance to be measured based on either or both of the level of agglutinate formation and the level of unagglutinated carrier particles after step (2) or (2').

2. The method of claim 1, wherein the three-dimensional information of agglutinates or carrier particles is physically measured in step (2) or (2').

3. The method of claim 2, wherein the method of physically measuring the three-dimensional information is a method selected from the group consisting of electric resistance method, laser diffraction/scattering method, and three-dimensional image analysis method.

4. The method of claim 1, wherein the voltage pulse is an alternating current voltage pulse.

5. The method of claim 1, which comprises counting the carrier particles after the electric field is removed in step (2) or (2').

6. The method of claim 5, which further comprises the step of diluting the carrier particles after the electric field is removed in step (2) or (2').

7. The method of claim 1, which comprises applying voltage pulses several times.

8. The method of claim 7, which comprises the step of applying voltage pulses, dispersing the carrier particles, and applying voltage pulses again.

9. The method of claim 7, wherein the voltage pulses are applied from different directions.

10. The method of claim 1, wherein the mean particle size of carrier particles is 1 $\mu$m or greater.

11. The method of claim 10, wherein the mean particle size of carrier particles is in the range of 1 to 20 $\mu$m.

12. A device for measuring an affinity substance, which comprises:

(a) a space containing carrier particles and an affinity substance to be measured, wherein the carrier particles are bound to a binding partner having an activity to bind to the affinity substance;

(b) electrodes for applying a voltage pulse to the carrier particles in the space; and

(c) a device for counting agglutinates formed through agglutination of carrier particles, or unagglutinated carrier particles in the space, or both, using their three-dimensional information as an indicator.

13. The device of claim 12, wherein the device of (c) is a means for physically measuring three-dimensional information.

14. The device of claim 13, wherein the device for physically measuring the three-dimensional information is a means for physically measuring three-dimensional information using a method selected from the group consisting of electric resistance method, laser diffraction/scattering method, and three-dimensional image analysis method.

15. The device of claim 12, which comprises at least two pairs of electrodes for applying the voltage pulse.

16. The device of claim 12, which comprises a means for moving the electrodes to apply the voltage pulse and which can supply an electric field from different directions with respect to the space.

(A)

SAMPLE REAGENT1 REAGENT2

(B)

# FIG. 1

(A)

(B) APPLYING PULSES

(C) AFTER BLOCKING PULSES
(IN THE ABSENCE OF ANTIGEN)

(D) AFTER BLOCKING PULSES
(IN THE PRESENCE OF ANTIGEN)

# FIG. 2

FIG. 3

FIG. 4

|  | DISTANCE BETWEEN ELECTRODES | VOLUME OF REACTION SOLUTION | REACTION VESSEL LENGTH |
|---|---|---|---|
|  | 0.5mm・・・ | 2.5 $\mu$ L | 10mm |
|  | 1.0mm・・・ | 10 $\mu$ L | 10mm |
|  | 0.5mm・・・ | 5 $\mu$ L | 20mm |
|  | 1.0mm・・・ | 20 $\mu$ L | 20mm |

FIG. 5

100 : LATEX PARTICLES
3 : ELECTRODE
3': ELECTRODE

FIG. 6

FIG. 7

FIG. 8

FIG. 9

## INTERNATIONAL SEARCH REPORT

| International application No. |
|---|
| PCT/JP2004/008553 |

A.  CLASSIFICATION OF SUBJECT MATTER
    Int.Cl$^7$  G01N33/543, G01N15/02, G01N15/12

According to International Patent Classification (IPC) or to both national classification and IPC

B.  FIELDS SEARCHED

Minimum documentation searched (classification system followed by classification symbols)
    Int.Cl$^7$  G01N33/543, G01N15/02, G01N15/12

Documentation searched other than minimum documentation to the extent that such documents are included in the fields searched
    Jitsuyo Shinan Koho          1922-1996   Toroku Jitsuyo Shinan Koho   1994-2004
    Kokai Jitsuyo Shinan Koho    1971-2004   Jitsuyo Shinan Toroku Koho   1996-2004

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y/ A | JP 07-083928 A   (Masao KARUBE), 31 March, 1995 (31.03.95), Full text (Family: none) | 1-8,10-14/ 9,15,16 |
| Y/ A | JP 01-259257 A   (Toa Medical Electronics Co., Ltd.), 16 October, 1989 (16.10.89), Claims; page 9, upper left column, line 1 to lower right column, line 15 & US 5215714 A          & EP 336013 A | 1-8,10-14/ 9,15,16 |
| Y/ A | JP 07-128217 A   (Toa Medical Electronics Co., Ltd.), 19 May, 1995 (19.05.95), Claims; Par. No. [0002] & US 5506673 A          & EP 652428 A | 1-8,10-14/ 9,15,16 |

☐  Further documents are listed in the continuation of Box C.          ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search    16 July, 2004 (16.07.04) | Date of mailing of the international search report    03 August, 2004 (03.08.04) |
|---|---|
| Name and mailing address of the ISA/    Japanese Patent Office | Authorized officer |
| Facsimile No. | Telephone No. |

Form PCT/ISA/210 (second sheet) (January 2004)